# EUROPEAN PATENT APPLICATION

(11) **EP 2 762 885 A1**
(43) Date of publication of application: **06.08.2014**
(21) Application number: 14153212.7
(22) Date of filing: 30.01.2014
(51) Int. Cl.: G01N 33/543, G01N 33/487

(54) **Immunoassay method and immunoassay apparatus**

(30) Priority: 31.01.2013 JP 2013017871
(71) Applicant: SYSMEX CORPORATION, Kobe-shi, Hyogo 651-0073 (JP)
(72) Inventor: Koshimura, Naoto, Hyogo, 651-0073 (JP)
(74) Representative: HOFFMANN EITLE

(57) **Abstract**

In order to provide an immunoassay method and an immunoassay apparatus capable of further reducing errors due to non-target substances, a detector measures a measurement specimen and detects information regarding the binding number of carrier particles included in the measurement specimen. A controller classifies, based on the information regarding the binding number, particles included in the measurement specimen into groups, the groups being classified in accordance with the binding numbers. Further, for each classified group, the controller performs either one of a first removing process for removing, from a processing target, data of non-target substances different from the carrier particles, and a second removing process for removing, from the processing target, data of the non-target substances through a process different from the first removing process, and obtains information regarding the agglutination degree of the carrier particles, based on data of the carrier particles obtained by performing the removing process.

## Description

### FIELD OF THE INVENTION

The present invention relates to immunoassay methods and immunoassay apparatuses using particle agglutination.

### BACKGROUND

To date, immunoassay methods using counting immunoassay (CIA) are known. In such an immunoassay method, a measurement specimen is prepared by mixing a sample and carrier particles having immobilized thereon an antigen or an antibody that corresponds to a measurement target substance, and the prepared measurement specimen is measured, whereby the agglutination degree of the carrier particles is obtained. In this assay method, if non-target substances such as chyle particles and the like are contained in the sample, an error occurs in a measurement result thereof. As a technology for reducing this error, the technologies described in, for example, U.S. Patent No. 5527714 and U.S. Patent Application Publication No. 2005/148099 are known.

In the technology described in U.S. Patent No. 5527714, in a particle size distribution chart whose horizontal axis represents particle size and whose vertical axis represents the number of particles, particle size distribution of non-target substances is estimated from data in a region in which carrier particles do not appear. Then, based on particle size distribution obtained by subtracting the estimated particle size distribution of the non-target substances from the entire particle size distribution, the agglutination degree is obtained.

In the technology described in U.S. Patent Application Publication No. 2005/148099, in a scattergram whose horizontal axis represents forward scattered light intensity and whose vertical axis represents side scattered light intensity or high-frequency resistance, a region in which carrier particles appear and a region in which non-target substances appear are set, and based on particles that have appeared in the region in which carrier particles appear, the agglutination degree is obtained.

The above two technologies are effective for reducing errors due to non-target substances such as chyle particles and the like, but it is desired that errors are further reduced.

### SUMMARY OF THE INVENTION

The scope of the present invention is defined solely by the appended claims, and is not affected to any degree by the statements within this summary.
(1) A first aspect of the present invention is an immunoassay method comprising:
   detecting, by measuring a measurement specimen obtained by mixing a sample and carrier particles together, information regarding a binding number of the carrier particles included in the measurement specimen, the carrier particles having immobilized thereon an antibody or an antigen against a measurement target substance;
   classifying, based on the information regarding the binding number, particles included in the measurement specimen into groups, the groups being classified in accordance with binding numbers; and
   obtaining information regarding an agglutination degree of the carrier particles based on data of the carrier particles obtained by performing, on at least one of the classified groups, at least one of a first removing process for removing, from a processing target, data of non-target substances different from the carrier particles, and a second removing process for removing, from the processing target, data of the non-target substances through a process different from the first removing process.
   According to the immunoassay method of this aspect, since an appropriate process for removing data of the non-target substances is applied to each classified group, influence of errors due to the non-target substances can be reduced.
(2) The immunoassay method according to (1), wherein
   the detecting step comprises:
      obtaining optical information from each particle included in the measurement specimen, by emitting light to the measurement specimen; and
      detecting forward scattered light information as the information regarding the binding number, based on the obtained optical information.
   As a result, the information regarding the binding number can be easily and accurately obtained.
(3) The immunoassay method according to (2), wherein
   the detecting step further comprises:
      detecting side scattered light information based on the obtained optical information; and
      the information obtaining step comprises:
         in the first removing process, removing data of the non-target substances from the processing target, based on the detected side scattered light information.
(4) The immunoassay method according to (2) or (3), wherein
   the information obtaining step comprises:
      in the second removing process, removing data of the non-target substances from the processing target, based on the detected forward scattered light information.
   As a result, in the first and second removing processes, the non-target substances can be easily and accurately removed from the processing target.
(5) The immunoassay method according to any one of (1) to (4), wherein
   the information obtaining step comprises:
      selecting, for each classified group, either one of the first removing process and the second removing process as a removing process appropriate for removing data of the non-target substances.
(6) The immunoassay method according to (5), wherein
   the information obtaining step comprises:
      selecting, for the classified group, either one of the first removing process and the second removing process as the removing process appropriate for removing data of the non-target substances, based on a magnitude of the binding number.
   As a result, an appropriate removing process can be assuredly applied to each classified group.
(7) The immunoassay method according to (5) or (6), wherein
   in the information obtaining step,
      the first removing process is performed when the first removing process has been selected for the classified group.
   Accordingly, the first removing process can be prevented from being unnecessarily performed, and thus, the removing process can be efficiently and quickly performed.
(8) The immunoassay method according to (5) or (6), wherein
   the information obtaining step comprises:
      performing, for each classified group, both of the first removing process and the second removing process; and
      obtaining information regarding the agglutination degree of the carrier particles, based on data of the carrier particles obtained by performing the removing process selected as the removing process appropriate for removing data of the non-target substances.
   According to this method, the removing process becomes slightly inefficient compared with the case where only one of the removing processes is performed as above. However, the information regarding the agglutination degree of the carrier particles is obtained, based on the data of the carrier particles obtained by the selected removing process as the removing process appropriate for removing data of the non-target substances. Therefore, as in the case above, advantageously, influence of errors due to the non-target substances can further be reduced.
(9) The immunoassay method according to any one of (1) to (8), wherein
   in the information obtaining step,
      with respect to a predetermined group among the classified groups, either one of the first removing process and the second removing process is selected in advance as a removing process appropriate for removing data of the non-target substances.
   In this case, preferably, when the binding number exceeds a predetermined binding number, the first removing process is selected, and when the binding number is less than or equal to a predetermined binding number, the second removing process is selected. This eliminates, with respect to a predetermined group, the need for performing a process for determining which of the first removing process and the second removing process is appropriate for removing data of the non-target substances, and thus, the processing can be simplified. It should be noted that, as also mentioned in the following embodiment, this method is preferred when, with respect to a predetermined group, it is clear which of the first removing process and the second removing process is more appropriate than the other for removing data of the non-target substances.
(10) The immunoassay method according to any one of (5) to (9), wherein
   the information obtaining step comprises:
      determining, with respect to a predetermined group among the classified groups, which of the first removing process and the second removing process is appropriate for removing data of the non-target substances; and
      selecting, based on a result of the determination, either one of the first removing process and the second removing process as the removing process appropriate for removing data of the non-target substances.
   Accordingly, with respect to the predetermined group, a more appropriate removing process is dynamically selected in accordance with the measurement specimen. Thus, the non-target substances can be more appropriately removed. It should be noted that, as also mentioned in the following embodiment, this method is preferred when, with respect to a predetermined group, it is not clear which of the first removing process and the second removing process is appropriate for removing data of the non-target substances.
(11) The immunoassay method according to (5), wherein
   in the information obtaining step,
      a determination condition for determining whether the first removing process is appropriate for removing data of the non-target substances is set in advance,
      the first removing process is selected for a group satisfying the determination condition, and
      the second removing process is selected for a group not satisfying the determination condition.
(12) The immunoassay method according to (10) or (11), wherein
   the information obtaining step comprises:
      in the first removing process, setting, for each classified group, a borderline for separating data of the carrier particles from data of the non-target substances, based on predetermined feature information representing a particle feature quantity; and
      determining, for each classified group, whether the first removing process is appropriate for removing data of the non-target substances, based on a number of particles containing the feature information in a predetermined range including the borderline.
   As a result, selection of an appropriate removing process for each classified group can be more assuredly and easily performed.
(13) The immunoassay method according to (12), wherein
   the information obtaining step comprises:
      determining, when a ratio of the number of particles containing the feature information in the predetermined range relative to a number of particles included in the classified group is less than or equal to a predetermined threshold value, the first removing process to be appropriate for removing data of the non-target substances.
   Accordingly, when the borderline between the carrier particles and the non-target substance is clear, the first removing process is selected. Thus, the non-target substances can be appropriately removed from the processing target.
(14) The immunoassay method according to (12) or (13), wherein
   the information obtaining step comprises:
      determining, when the number of particles containing the feature information in the predetermined range is less than or equal to a predetermined threshold value, the first removing process to be appropriate for removing data of the non-target substances.
   Also in this case, when the borderline between the carrier particles and the non-target substance is clear, the first removing process is selected. Thus, the non-target substances can be appropriately removed from the processing target.
(15) The immunoassay method according to any one of (12) to (14), wherein
   the detecting step comprises:
      obtaining optical information from each particle included in the measurement specimen, by emitting light to the measurement specimen; and
      obtaining side scattered light information as the feature information, based on the obtained optical information.
   As the feature information, in addition to side scattered light information, high-frequency resistance can also be used as in Patent Literature 2 above.
(16) The immunoassay method according to any one of (1) to (15), wherein
   in the information obtaining step,
      the second removing process includes a process of estimating a state of distribution of the non-target substances, and a process of subtracting the estimated distribution of the non-target substances from distribution of particles included in each classified group.
   Accordingly, in a group for which the second removing process is appropriate, influence of errors due to the non-target substances can be further reduced.
(17) The immunoassay method according to (9), wherein
   in the information obtaining step,
      with respect to a predetermined group among the classified groups, when the binding number exceeds a predetermined binding number, the first removing process is selected, and when the binding number is less than or equal to a predetermined binding number, the second removing process is selected.
   This eliminates, with respect to a predetermined group, the necessity of performing process for determining which of the first removing process and the second removing process is appropriate for removing data of the non-target substances, and thus, the processing can be simplified.
(18) A second aspect of the present invention is an immunoassay apparatus comprising:
   a detector configured to detect, by measuring a measurement specimen obtained by mixing a sample and carrier particles together, information regarding a binding number of the carrier particles included in the measurement specimen, the carrier particles having immobilized thereon an antibody or an antigen against a measurement target substance; and
   a controller configured to perform operations comprising:
      classifying, based on the information regarding the binding number, particles included in the measurement specimen into groups, the groups being classified in accordance with the binding numbers;
      performing, on at least one of the classified groups, at least one of a first removing process for removing, from a processing target, data of non-target substances different from the carrier particles, and a second removing process for removing, from the processing target, data of the non-target substances through a process different from the first removing process; and
      obtaining information regarding an agglutination degree of the carrier particles, based on data of the carrier particles obtained by performing the at least one removing process.
   According to an immunoassay apparatus of this aspect, as in the immunoassay method of the first aspect above, an appropriate process for removing data of the non-target substances is applied to each classified group in accordance with the binding number of the carrier particles. Thus, influence of errors due to the non-target substances can further be reduced.
(19) The immunoassay apparatus according to (18), wherein
   the controller selects, for each classified group, either one of the first removing process and the second removing process as a removing process appropriate for removing data of the non-target substances.
(20) The immunoassay apparatus according to (19), wherein
   the controller selects, for the classified group, either one of the first removing process and the second removing process as the removing process appropriate for removing data of the non-target substances, based on a magnitude of the binding number.
(21) The immunoassay apparatus according to any one of (18) to (20), wherein
   the detector
      comprises a light source configured to emit light to the measurement specimen, and a light receiving part configured to receive light from each particle included in the measurement specimen, and
      detects forward scattered light information as the information regarding the binding number, based on information of light received by the light receiving part.
(22) The immunoassay apparatus according to (21), wherein
   the detector further detects side scattered light information, based on information of light received by the light receiving part.
(23) A third aspect of the present invention is an immunoassay method comprising:
   detecting, by measuring a measurement specimen obtained by mixing a sample and carrier particles together, information regarding a binding number of the carrier particles included in the measurement specimen, the carrier particles having immobilized thereon an antibody or an antigen against a measurement target substance;
   classifying, based on the information regarding the binding number, particles included in the measurement specimen into groups, the groups being classified in accordance with the binding numbers; and
   obtaining information regarding an agglutination degree of the carrier particles based on data of the carrier particles obtained by performing, on at least one of the classified groups, at least one of a first process for identifying data of the carrier particles from among data of particles included the at least one classified group, and a second process for identifying data of the carrier particles from among data of particles included the at least one classified group through a process different from the first process.
(24) A fourth aspect of the present invention is an immunoassay apparatus comprising:
   a detector configured to detect, by measuring a measurement specimen obtained by mixing a sample and carrier particles together, information regarding a binding number of the carrier particles included in the measurement specimen, the carrier particles having immobilized thereon an antibody or an antigen against a measurement target substance; and
   a controller configured to perform operations comprising:
      classifying, based on the information regarding the binding number, particles included in the measurement specimen into groups, the groups being classified in accordance with the binding numbers;
      performing, on at least one of the classified groups, at least one of a first process for identifying data of the carrier particles from among data of particles included the at least one classified group, and a second removing process for identifying data of the carrier particles from among data of particles included the at least one classified group through a process different from the first process; and
      obtaining information regarding an agglutination degree of the carrier particles, based on data of the carrier particles obtained by performing the at least one removing process.

### BRIEF DESCRIPTION OF THE DRAWINGS

FIG. 1A shows an external view of a structure of an immunoassay apparatus;
FIG. 1B shows an internal structure of the immunoassay apparatus;
FIG. 2A is a schematic view of a structure of a specimen preparing unit;
FIG. 2B is a schematic view of a structure of a measurement unit;
FIG. 2C is a schematic view of a structure of a sheath flow cell;
FIG. 3 is a block diagram showing a configuration of the immunoassay apparatus;
FIG. 4 is a flow chart showing processing performed by a controller;
FIG. 5A illustrates a scattergram regarding each particle using forward scattered light intensity and side scattered light intensity as parameters;
FIG. 5B shows demarcation lines set in the scattergram in FIG. 5A;
FIG. 6A illustrates a histogram of each particle using forward scattered light intensity and the number of particles as parameters;
FIG. 6B shows values used in setting demarcation lines;
FIG. 7 is a flow chart showing a counting process;
FIG. 8A is a flow chart showing a selecting process;
FIG. 8B is a flow chart showing a specific process of S105 in FIG. 7;
FIG. 9A to FIG. 9D are schematic views for describing the procedure of the counting process;
FIG. 10A illustrates a histogram used for estimating a curve representing distribution of non-target substances;
FIG. 10B illustrates a histogram showing distribution of non-agglutinated particles and agglutinated particles being target particles;
FIG. 11 shows results of calculation of the concentration of measurement target substances actually performed by the immunoassay apparatus of the present embodiment;
FIG. 12 is a flow chart showing a counting process of modification 1;
FIG. 13A is a flow chart showing a counting process of modification 2;
FIG. 13B shows a table used in the counting process of the modification 2;
FIG. 14A is a flow chart showing a counting process of modification 3; and
FIG. 14B is a table used in the counting process of the modification 3.

### DETAILED DESCRIPTION OF THE PREFERRED EMBODIMENTS

The preferred embodiments of the present invention will be described hereinafter with reference to the drawings.

The present embodiment is obtained by applying the present invention to an immunoassay method and an immunoassay apparatus for detecting, by use of a particle agglutination method, agglutination occurring as a result of a measurement target substance and carrier particles being mixed together, and for quantifying the measurement target substance based on a result obtained from the detection.

In the particle agglutination method, a sample containing a measurement target substance, and carrier particles having immobilized thereon an antibody or an antigen corresponding to the measurement target substance are mixed together. If the measurement target substance is present in the sample, carrier particles agglutinate as a result of antigen-antibody reaction. As an antibody or an antigen to be immobilized on carrier particles, in a case where the measurement target substance is an antibody, an antigen that specifically reacts with the antibody through antigen-antibody reaction is used; and in a case where the measurement target substance is an antigen, an antibody that specifically reacts with the antigen through antigen-antibody reaction is used. For example, in a case where the measurement target substance is carcinoembryonic antigen (CEA antigen), an anti-CEA antibody is immobilized on carrier particles. As carrier particles, those generally used in particle agglutination methods, such as latex particles, metal particles, and dendrimers, are used, for example.

Hereinafter, an immunoassay apparatus 1 according to the present embodiment will be described with reference to the drawings.

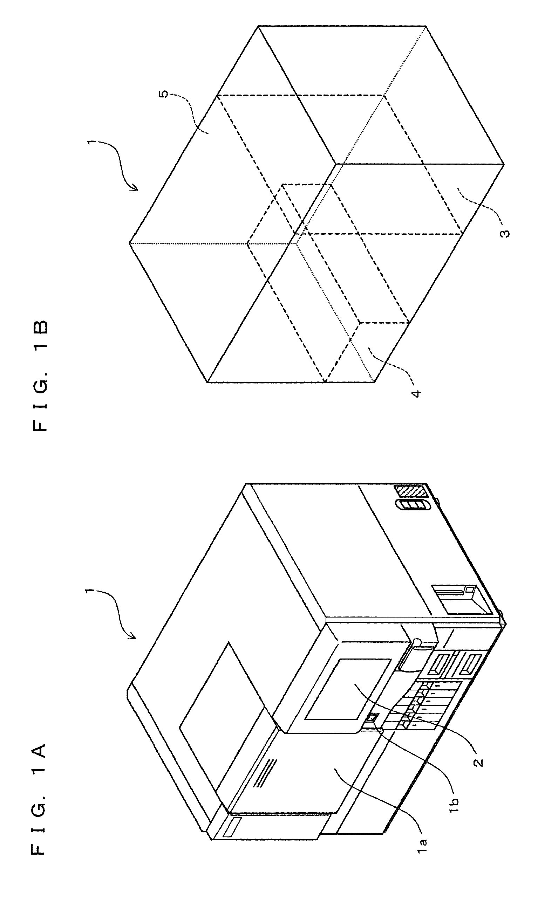

FIG. 1A shows an external view of a structure of the immunoassay apparatus 1. FIG. 1B shows an internal structure of the immunoassay apparatus 1.

On the front face of the immunoassay apparatus 1, a cover 1a, a start switch 1b, and a display input unit 2 including a touch panel are arranged. In the right space within the immunoassay apparatus 1, a controller 3 for controlling components is arranged. In the lower left space within the immunoassay apparatus 1, a measurement unit 4 for detecting signals from a measurement specimen is arranged. In the other space within the immunoassay apparatus 1, a specimen preparing unit 5 for preparing a measurement specimen is arranged.

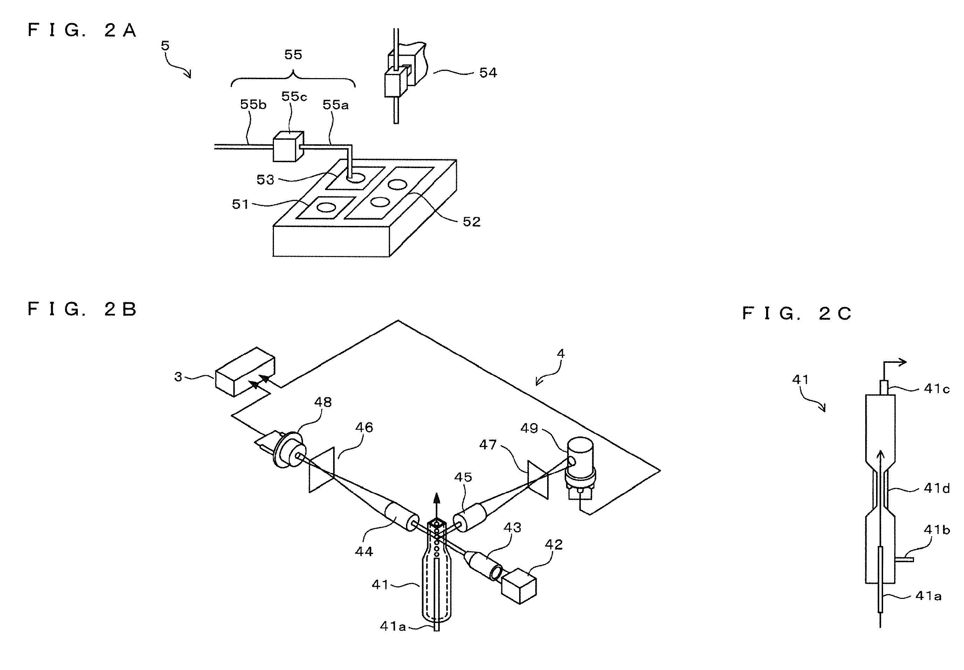

FIG. 2A is a schematic view of a structure of the specimen preparing unit 5.

The specimen preparing unit 5 includes a sample setting part 51, a reagent setting part 52, a reaction part 53, a dispensing apparatus 54, and a liquid sending apparatus 55. A user can open the cover 1a to set various containers in the sample setting part 51 and the reagent setting part 52. In the sample setting part 51, a container containing a sample, such as blood or urine which contains a measurement target substance, is set. In the reagent setting part 52, a container containing a carrier particle suspension and a container containing a reaction buffer solution are set. The carrier particle suspension is obtained by suspending carrier particles in an appropriate liquid such as water or a buffer solution. The reaction buffer solution is for adjusting the environment for antigen-antibody reaction to occur, by being added to the sample together with the carrier particle suspension. In the reaction part 53, a vacant cuvette is set.

The dispensing apparatus 54 includes a tube for aspirating and discharging a liquid by a predetermined amount from the tip thereof, and is configured to be able to move in the up-down, left-right, and forward-backward directions by means of a driving device (not shown). By the dispensing apparatus 54, a sample, the carrier particle suspension, and the reaction buffer solution are dispensed into a cuvette in the reaction part 53, as appropriate. The reaction part 53 includes a temperature adjusting mechanism (not shown) for keeping constant the temperature of the solution in the cuvette, and an agitation mechanism (not shown) for agitating the solution in the cuvette. Within the cuvette set in the reaction part 53, the sample, the carrier particle suspension, and the reaction buffer solution are mixed together, whereby a measurement specimen is prepared.

The liquid sending apparatus 55 includes an aspiration tube 55a for aspirating the measurement specimen, a liquid sending tube 55b for sending the measurement specimen aspirated by the aspiration tube 55a to the measurement unit 4, and a pump 55c for aspirating the measurement specimen and sending the aspirated measurement specimen to the measurement unit 4. The liquid sending apparatus 55 is configured to be able to move in the up-down, left-right, and forward-backward directions by means of a driving device (not shown). By the liquid sending apparatus 55, the measurement specimen in the cuvette set in the reaction part 53 is sent to the measurement unit 4.

FIG. 2B is a schematic view of a structure of the measurement unit 4. FIG. 2C is a schematic view of a structure of a sheath flow cell 41.

The measurement unit 4 includes a flow cell 41, a laser light source 42, a condenser lens 43, and condensing lenses 44 and 45, pinholes 46 and 47, a photodiode 48, and a photomultiplier tube 49. The flow cell 41 is for flowing the measurement specimen prepared in the specimen preparing unit 5, being surrounded by a sheath fluid. As shown in FIG. 2C, the flow cell 41 includes a specimen nozzle 41a which upwardly jets the measurement specimen toward a pore part 41d, a sheath fluid inlet 41b, and a waste liquid outlet 41c.

A laser beam emitted from the laser light source 42 advances through the condenser lens 43 to be applied on the pore part 41d of the flow cell 41. Accordingly, the measurement specimen passing through the pore part 41d is irradiated with the laser beam. The condensing lenses 44 and 45 respectively condense forward scattered light and side scattered light obtained from each particle in the measurement specimen irradiated with the laser beam. The photodiode 48 receives forward scattered light that has passed through the pinhole 46, and performs opto-electric conversion on the received forward scattered light to generate a forward scattered light signal. The photomultiplier tube 49 receives side scattered light that has passed through the pinhole 47, and performs opto-electric conversion on the received side scattered light to generate a side scattered light signal. The forward scattered light signal and the side scattered light signal are generated for each particle in the measurement specimen, and the generated forward scattered light signal and side scattered light signal are sent to the controller 3.

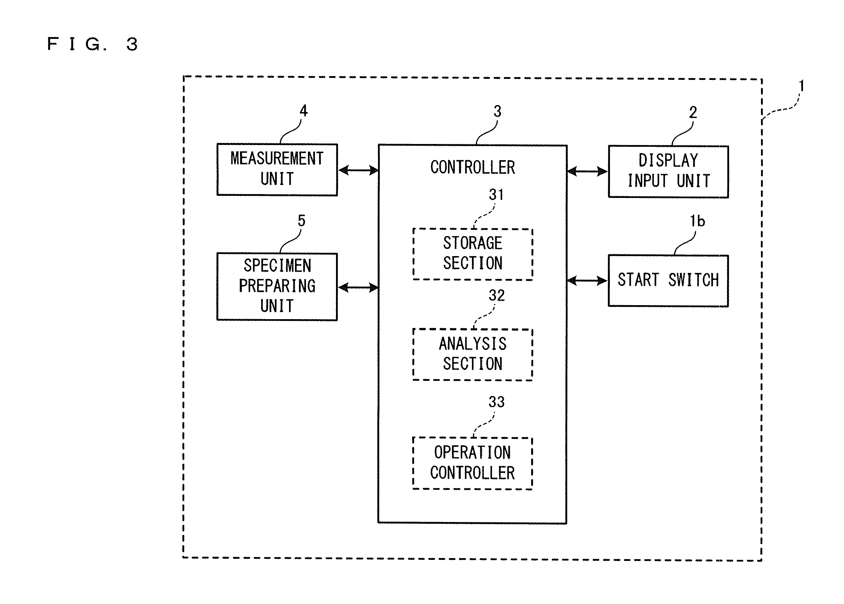

Based on the received forward scattered light signal and side scattered light signal, the controller 3 calculates an forward scattered light intensity and a side scattered light intensity, respectively, and stores these intensities in a storage section 31 (see FIG. 3).

Here, the number of carrier particles included in each particle that has passed through the pore part 41d of the flow cell 41 will be referred to as a "binding number". A carrier particle that has not yet been agglutinated, that is, a particle whose binding number is 1, will be referred to as "non-agglutinated particle", and an aggregate resulting from the antigen-antibody reaction between the measurement target substance and the carrier particles, that is, a particle whose binding number is 2 or more, will be referred to as an "agglutinated particle". Among such agglutinated particles, particles whose binding number is 2, 3, and 4 will be respectively referred to as a "2-agglutinated particle", a "3-agglutinated particle", and a "4-agglutinated particle", and particles whose binding number is 5 or more will be collectively referred to as a "particle whose binding number is 5" or a "5-agglutinated particle". The sizes of particles whose binding numbers are 1 to 5, i.e., a non-agglutinated particle, a 2-agglutinated particle, a 3-agglutinated particle, a 4-agglutinated particle, and a 5-agglutinated particle, become larger in this order. Since the size of a particle changes in accordance with its binding number in this manner, the controller 3 can determine, based on the magnitude of a forward scattered light intensity, to which of the five classifications a particle that has passed through the pore part 41 d of the flow cell 41 belongs. The controller 3 can also count non-agglutinated particles and agglutinated particles separately from each other, and thus, can determine the agglutination degree. As the agglutination degree of the present embodiment, the value of P/T, which is calculated based on the number of non-agglutinated particles (M), the number of agglutinated particles (P), and the total sum number of particles (T) being a total of M and P, is used.

FIG. 3 is a block diagram showing a configuration of the immunoassay apparatus 1.

The controller 3 includes: a microcomputer which includes a CPU and a storage device such as a ROM, a RAM, and the like; a circuit which processes various signals; and the like. Accordingly, the controller 3 has functions of the storage section 31, an analysis section 32, and an operation controller 33.

The storage section 31 has stored therein: a program for calculating a forward scattered light intensity and a side scattered light intensity based on a forward scattered light signal and a side scattered light signal received from the measurement unit 4; an analysis program for analyzing the measurement specimen based on the forward scattered light intensity and the side scattered light intensity; and a control program for controlling operation of components of the apparatus. The storage section 31 further stores therein data of the calculated forward scattered light intensity and side scattered light intensity, and analysis results obtained by the analysis program; and further stores therein contents of various settings. The analysis section 32 analyzes the measurement specimen based on the analysis program and generates an analysis result regarding the measurement specimen. The analysis result generated in the analysis section 32 is outputted to the display input unit 2. The operation controller 33 controls operation of components of the apparatus based on the control program stored in the storage section 31.

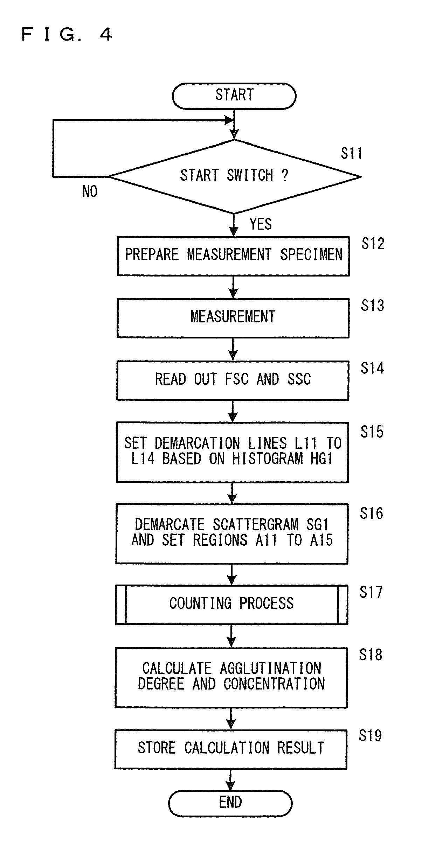

FIG. 4 is a flow chart showing processing performed by the controller 3.

Before the processing shown in FIG. 4 is performed, first, the user opens the cover 1 a and sets a container containing a sample and containers each containing a reagent, in the sample setting part 51 and the reagent setting part 52, respectively. Then, the user presses the start switch 1b to start the processing.

Upon the start switch 1b being pressed (S11: YES), the controller 3 causes a measurement specimen to be prepared (S12). Specifically, the dispensing apparatus 54 dispenses the sample contained in the container set in the sample setting part 51, into a cuvette set in the reaction part 53 by a predetermined amount (for example, 10 µL). Subsequently, the dispensing apparatus 54 dispenses the reaction buffer solution contained in the container set in the reagent setting part 52, into the cuvette set in the reaction part 53 by a predetermined amount (for example, 80 µL), and dispenses the carrier particle suspension contained in the container set in the reagent setting part 52, into the cuvette set in the reaction part 53 by a predetermined amount (for example, 10 µL). Then, the controller 3 causes the reaction part 53 to agitate the specimen in the cuvette for a predetermined time period (for example, 5 minutes) while maintaining the temperature of the cuvette at a predetermined temperature (for example, 45°C). Accordingly, a measurement specimen is prepared in the cuvette. Then, the liquid sending apparatus 55 sends the measurement specimen in the cuvette of the reaction part 53 by a predetermined amount, to the flow cell 41 of the measurement unit 4.

Subsequently, the controller 3 causes the measurement unit 4 to perform measurement (S13). Specifically, the measurement unit 4 generates a forward scattered light signal and a side scattered light signal based on forward scattered light and side scattered light obtained from each particle in the measurement specimen. Based on the forward scattered light signal and the side scattered light signal, the controller 3 calculates a forward scattered light intensity and a side scattered light intensity, respectively, and stores these intensities in the storage section 31. Subsequently, the controller 3 reads out the forward scattered light intensity and the side scattered light intensity stored in the storage section 31 (S14), and starts analysis of the measurement specimen.

Hereinafter, scattergrams and histograms will be referred to as appropriate for convenience for description. These scattergrams and histograms not necessarily need to be created as figures or graphs, and it is sufficient that similar results can be obtained through data processing. Further, in the steps of creating these scattergrams and histograms, graphs each using two axes not necessarily need to be created. It is sufficient that data having data structure equivalent to these scattergrams and histograms is created based on data of the forward scattered light intensity and the side scattered light intensity stored in the storage section 31.

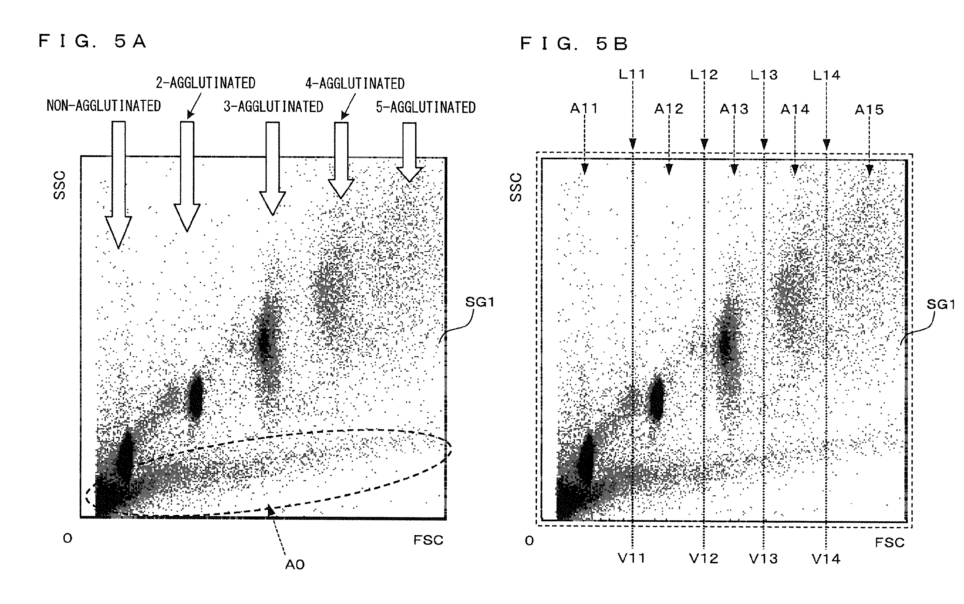

FIG. 5A illustrates a scattergram SG1 using, as parameters, forward scattered light intensity (hereinafter, referred to as "FSC") and side scattered light intensity (hereinafter, referred to as "SSC") of each particle read out in S14 in FIG. 4. The horizontal axis represents FSC, and the vertical axis represents SSC. FSC is information reflecting the size of a particle, and the size of a particle increases rightward in the scattergram SG1. SSC is information reflecting the internal information (density) of a particle and the internal information (density) of a particle increases upward in the scattergram SG1.

In the scattergram SG1, particles whose binding numbers are 1 to 5, i.e., non-agglutinated particles, 2-agglutinated particles, 3-agglutinated particles, 4-agglutinated particles, and 5-agglutinated particles are distributed from lower left to upper right, in this order. In this manner, in the scattergram SG1, distribution of the non-agglutinated particles and distributions of the agglutinated particles are different from one another in accordance with their binding numbers. Therefore, if a region in which each type of particle is distributed is set, particles can be counted separately by type.

With reference back to FIG. 4, the controller 3 sets, as shown in FIG. 5B, demarcation lines L11 to L14 for demarcating the scattergram SG1 into regions A11 to A15 in which non-agglutinated particles and 2- to 5-agglutinated particles are respectively distributed (S15). The demarcation lines L11 to L14 are set based on a histogram HG1 as described below.

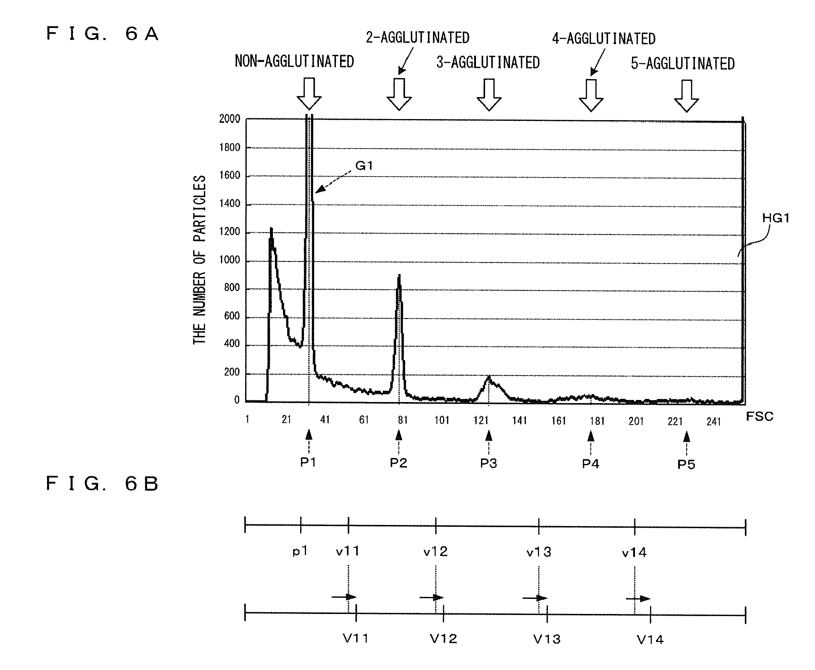

FIG. 6A illustrates the histogram HG1 using, as parameters, FSC of each particle read out in S 14 shown in FIG. 4 and the number of particles. The horizontal axis represents FSC and the vertical axis represents the number of particles. As shown in a curve G1 in the histogram HG1, particles whose binding numbers are 1 to 5, i.e., non-agglutinated particles, 2-agglutinated particles, 3-agglutinated particles, 4-agglutinated particles, and 5-agglutinated particles, are distributed near FSC values (peak values P1 to P5) where the numbers of particles peak, respectively. Therefore, the demarcation lines L11 to L14 for providing demarcation for the regions A11 to A15 shown in FIG. 5B are set between adjacent peak values, respectively.

The demarcation lines L11 to L14 maybe fixed to positions which are empirically and statistically considered as appropriate. However, the demarcation lines L11 to L14 tend to be slightly shifted for each measurement specimen even among samples of the same type. Therefore, in order to increase demarcation accuracy for the regions A11 to A15, it is preferable that the demarcation lines L11 to L14 are finely adjusted for each measurement specimen. Thus, in the present embodiment, fixed values v11 to v14 of the demarcation lines L11 to L14, the fixed values being empirically and statistically considered as appropriate, are held in advance, and these fixed values v11 to v14 are each adjusted in accordance with a peak position (peak value P1) of non-agglutinated particles in the measurement specimen, whereby values V11 to V14 specifying the demarcation lines L11 to L14 are determined.

In the storage section 31, as shown in FIG. 6B, an FSC fixed value p1, which is empirically and statistically determined as the peak of non-agglutinated particles, is stored. Further, in the storage section 31, FSC fixed values v11 to v14 are stored. These FSC fixed values v11 to v14 are empirically and statistically determined as distribution borderlines, respectively, between: non-agglutinated particles and 2-agglutinated particles; 2-agglutinated particles and 3-agglutinated particles; 3-agglutinated particles and 4-agglutinated particles; and 4-agglutinated particles and 5-agglutinated particles.

When the histogram HG1 is in the state shown in FIG. 6A, the controller 3 multiplies the fixed values v11 to v14 with a value (P1/p1) obtained by dividing the peak value P1 of this case by the fixed value p1, thereby calculating the values V11 to V 14. That is, the values V11 to V14 are v11× (P1/p1), v12 × (P1/p1), v13 × (P1/p1), and v14 × (P1/p1), respectively. The controller 3 sets the demarcation lines L11 to L14 extending in the up-down direction as shown in FIG. 5B, in accordance with the calculated values V11 to V14, respectively.

In the storage section 31, not only the FSC fixed value p1 empirically and statistically determined as the peak of non-agglutinated particles, but also FSC fixed values p2 to p5, which are empirically and statistically determined as the peaks of the 2- to 5-agglutinated particles, respectively, may be stored. In this case, for example, the values V11 to V14 can be determined as v11 × (P1/p1), v12 × (P2/p2), v13 × (P3/p3), and v14 × (P4/p4), respectively.

With reference back to FIG. 4, subsequently, the controller 3 demarcates the entire region of the scattergram SG1 by the demarcation lines L11 to L14 as shown in FIG. 5B, and sets the regions A11 to A15 in which particles whose binding numbers are 1 to 5 are respectively distributed (S16). Then, the controller 3 performs a counting process for counting the number of non-agglutinated particles and 2- to 5-agglutinated particles respectively included in the regions A11 to A 15 (S 17).

In the lower part of the scattergram SG1, for example, as shown in a region A0 in FIG. 5A, there may be a case where non-target substances such as chyle particles and the like are distributed. In such a case, in the regions A11 to A15 in FIG. 5B, non-target substances are included along with non-agglutinated particles or agglutinated particles. Therefore, if simply setting the regions A11 to A15 corresponding to their binding numbers and counting the particles included in the regions A11 to A15, non-agglutinated particles and agglutinated particles included in the regions A11 to A15 cannot be accurately counted.

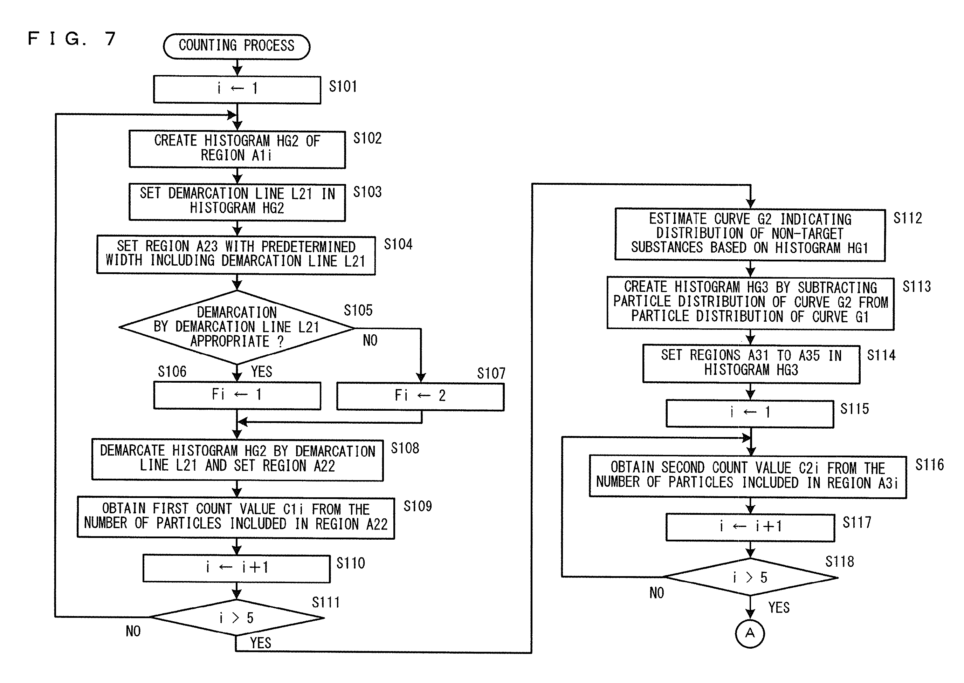

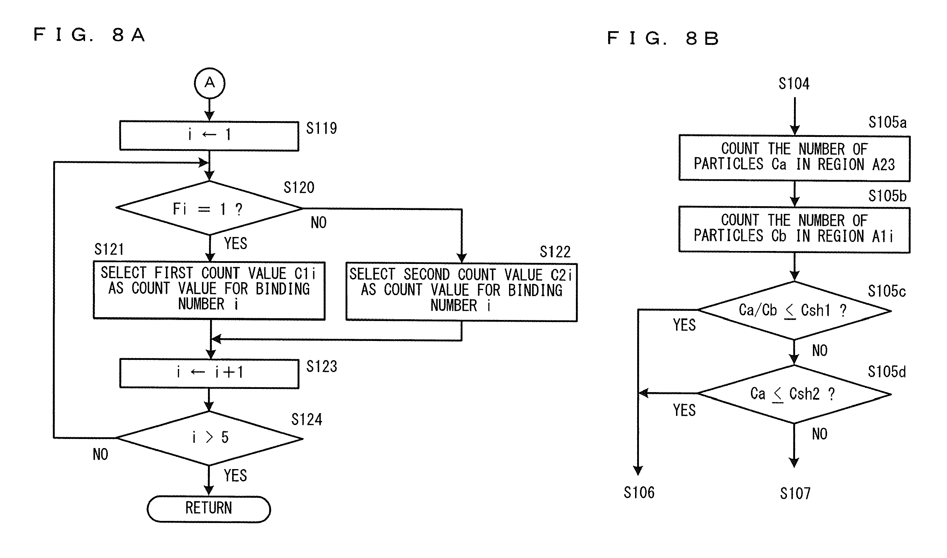

Therefore, in the present embodiment, by the counting process (S 17) shown in FIG. 4, non-target substances included in the regions A11 to A15 are removed from the counting target, whereby each type of particles whose binding numbers are 1 to 5 are accurately counted. The counting process (S 17) will be described with reference to FIG. 7 and FIG. 8A later.

With reference back to FIG. 4, next, the controller 3 calculates an agglutination degree and a concentration based on the number of particles counted by type (S 18). Specifically, the controller 3 totals the count values of 2- to 5-agglutinated particles obtained through the counting process to obtain a total number of particles (P), and then adds the number of particles (M) of non-agglutinated particles to the total number of particles (P) to obtain a total sum number of particles (T). Then, the controller 3 calculates an agglutination degree (P/T) from the obtained total number of particles (P) and the obtained total sum number of particles (T), and further calculates a concentration of the measurement target substance included in this sample, based on the calculated agglutination degree and a calibration curve created in advance.

Subsequently, the controller 3 stores the result calculated in S 18, in the storage section 31 (S 19). The calculated concentration of the measurement target substance is displayed on the display input unit 2 (see FIG. 1A) of the immunoassay apparatus 1 as appropriate. Then, the processing performed by the controller 3 ends.

FIG. 7 is a flow chart showing the counting process (S 17).

First, the controller 3 sets 1 to a variable i stored in the storage section 31 (S101). Next, the controller 3 performs processes of S 102 to S 109, on a region A1i (here, the region is the region A11 because the variable i is 1) shown in FIG. 5B. That is, the controller 3 performs, on the region A11, a first removing process (S102, S103, and S108) for removing non-target substances from the counting target, and a process (S109) for obtaining a first count value C11 by counting the remaining particles after the non-target substances have been removed. At this time, the controller 3 further performs processes (S104 to S107) for determining whether the first removing process is appropriate for removing data of the non-target substances, and setting a value corresponding to the appropriateness/inappropriateness to a flag F1.

When the processes onto the region A11 have ended, the controller 3 adds 1 to the variable i (S110), and determines whether the value of the variable i after the addition of 1 has exceeded 5 (S111). When the variable i has not exceeded 5 (S111: NO), the controller 3 returns to S102 and performs the processes of S102 to S109 on the region A1i. Here, since the value of the variable i is 2, the processes of S102 to S109 are performed on the region A12 in FIG. 5B, and a first count value C12 and a flag F2 are obtained.

Then, the controller 3 repeats the processes of S102 to S109 until the value of the variable i exceeds 5. As a result, with respect to the regions A13, A14, and A15 in FIG. 5B, first count values C13, C 14, and C 15, and flags F3, F4, and F5 are obtained, respectively.

Hereinafter, specific processes of S102 to S109 will be described with reference to exemplary processing performed on the region A13 (variable i = 3) in FIG. 5B. It should be noted that the processing onto the regions A11 (variable i =1), A12 (variable i = 2), A14 (variable i = 4), and A15 (variable i = 5) in FIG. 5B is also performed in the steps similar to those described below.

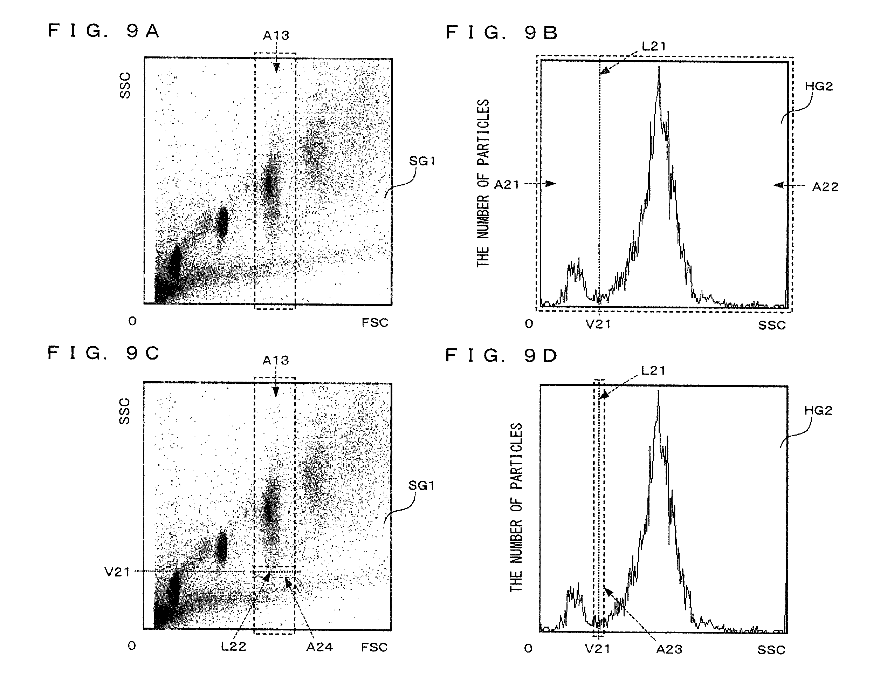

FIG. 9A to FIG. 9D are schematic views showing the procedure of the processing performed onto the region A13.

The controller 3 creates a histogram HG2 using SSC and the number of particles as parameters, with respect to the particles included in the region A13 of the scattergram SG1 shown in FIG. 9A (S102). Next, with respect to the histogram HG2, the controller 3 sets a demarcation line L21 for separating target particles (in this case, 3-agglutinated particles) from non-target substances included in the region A13 (S103).

FIG. 9B shows the waveform of the histogram HG2. In the histogram HG2, the left peak portion corresponds to non-target substances, and the right peak portion corresponds to 3-agglutinated particles. In S 102 of FIG. 7, the controller 3 first performs a smoothing process on the waveform of the histogram HG2, to smooth the waveform of the histogram HG2. Then, the controller 3 obtains an SSC value V21 where the number of particles becomes smallest between the peak of the left peak portion and the peak of the right peak portion. Then, the controller 3 sets the demarcation line L21 extending in the up-down direction, in accordance with the obtained value V21. The demarcation line L21 corresponds to a demarcation line L22 on the scattergram SG1 as shown in FIG. 9C.

Subsequently, in order to determine whether the demarcation between the target particles and the non-target substances by the demarcation line L21 is appropriate, the controller 3 sets a region A23 having a predetermined width including the demarcation line L21 (S104). That is, as shown in FIG. 9D, the controller 3 sets the region A23 having a predetermined range in each of a direction in which SSC decreases and a direction in which SSC increases, relative to the demarcation line L21. The region A23 corresponds to a region A24 on the scattergram SG1 as shown in FIG. 9C.

Here, if the number of particles included in the region A23 is small, it means that the number of the target particles and the non-target substances existing near the demarcation line L21 is small. Thus, the controller 3 determines that, in the region A13, the separation between the region corresponding to the target particles and the region of the non-target substances is good. In this case, the controller 3 determines that the demarcation between the target particles and the non-target substances by the demarcation line L21 is appropriate. On the other hand, if the number of particles included in the region A23 is large, it means that the target particles and the non-target substances exist in a mixed state near the demarcation line L21. Thus, the controller 3 determines that, in the region A13, the separation between the region corresponding to the target particles and the region of the non-target substances is not good. In this case, the controller 3 determines that the demarcation between the target particles and the non-target substances by the demarcation line L21 is not appropriate.

In this manner, the controller 3 determines whether the demarcation between the target particles and the non-target substances by the demarcation line L21 is appropriate, based on the number of particles included in the region A23 having a predetermined width including the demarcation line L21 (S105). Then, when the demarcation by the demarcation line L21 is appropriate (S105: YES), the controller 3 sets 1 to the flag F3 (S106), and when the demarcation by the demarcation line L21 is not appropriate (S105: NO), the controller 3 sets 2 to the flag F3 (S107).

FIG. 8B shows the specific process of S105. The controller 3 counts the number of particles Ca included in the region A23 (S105a), and further counts the number of particles Cb included in the region A13 (variable i = 3) (105b). Then, the controller 3 determines whether the ratio Ca/Cb of the number of particles Ca relative to the number of particles Cb is less than or equal to a predetermined threshold value Csh1 (S105c). When the ratio Ca/Cb is less than or equal to the threshold value Csh1 (S105c: YES), the controller 3 determines that the demarcation by the demarcation line L21 is appropriate and sets 1 to a flag Fi in S106 in FIG. 7. On the other hand, when the ratio Ca/Cb is not less than or equal to the threshold value Csh1 (S105c: NO), the controller 3 further determines whether the number of particles Ca included in the region A23 is less than or equal to a predetermined threshold value Csh2 (S105d). When the number of particles Ca is less than or equal to the threshold value Csh2 (S105d: YES), the controller 3 determines that the demarcation by the demarcation line L21 is appropriate and sets 1 to the flag Fi in S106 in FIG. 7. When the number of particles Ca is not less than or equal to the threshold value Csh2 (S105d: NO), the controller 3 determines that the demarcation by the demarcation line L21 is not appropriate, and sets 2 to the flag Fi in S107 in FIG. 7.

With reference back to FIG. 7, after determining whether the demarcation between the target particles and the non-target substances by the demarcation line L21 is appropriate, the controller 3 demarcates the histogram HG2 by the demarcation line L21 as shown in FIG. 9B and sets a region A22 which does not include a region A21 in which the non-target substances are distributed, that is, the region A22 in which the target particles are distributed (S 108). Then, the controller 3 counts the number of particles included in the region A22 and obtains the count value as the first count value C13 for the region A13 (S109).

It should be noted that, in the present embodiment, as shown in the flow chart in FIG. 7, when it has been determined that the demarcation by the demarcation line L21 is appropriate in S105 (S105: YES), and also when it has been determined that the demarcation by the demarcation line L21 is not appropriate (S105: NO), the region A22 is set in S108 and the first count value C 13 is obtained in S109.

In this manner, the first count value C13 for the region A13 is obtained and the flag F3 is set. Also with respect to the other regions A11, A12, A14, and A15, the processes of S102 to S109 are performed, whereby the demarcation line L21 is set in the same manner as described above, the first count values C11, C12, C14, and C 15 are obtained, and further, the flags F1, F2, F4, and F5 are set.

In the processes of S102 to S109 in FIG. 7, the region A21 in which the non-target substances are distributed is removed from the processing target through the process of S 108. Thus, a first count value C1i obtained in S109 is a value for which influence of the non-target substances is suppressed. However, as shown in FIG. 5A and FIG. 5B, in regions with large binding numbers, such as the regions A14 and A15 corresponding to 4-agglutinated particles and 5-agglutinated particles, the non-target substances are clearly separated from the target particles, but in accordance with decrease of the binding number, the borderline between the non-target substances and the target particles gradually becomes unclear. Therefore, with respect to regions with large binding numbers (regions A14, A15, and the like), the first removing process (S102, S103, and S108) in FIG. 7 can accurately remove the non-target substances, but with respect to regions with small binding numbers (region A11, A12, and the like), the accuracy of removing data of the non-target substances by the first removing process is decreased.

In the flow chart in FIG. 7, whether the first removing process (S102, S103, and S 108) is appropriate for removing data of the non-target substances for the region A1i is shown by the value of the flag Fi set in S 106 or S 107. That is, when it has been determined that the demarcation by the demarcation line L21 is appropriate in S 105 (S 105: YES), 1 is set to the flag Fi so as to indicate that the first removing process is appropriate. On the other hand, when it has been determined that the demarcation by the demarcation line L21 is not appropriate in S105 (S105: NO), 2 is set to the flag Fi so as to indicate that the first removing process is not appropriate. The flag Fi set in this manner is referred to when determining which of the first count value C1i and a second count value C2i obtained through S112 to S118 is to be selected as a count value of the target particles for the region A1i.

Upon completion the above processing, the controller 3 performs a second removing process (S112 to S114) for removing the non-target substances from the counting target, and processes (S115 to S118) for obtaining second count values C21 to C25 which are count values of particles whose binding numbers are 1 to 5, by counting remaining particles after the non-target substances has been removed.

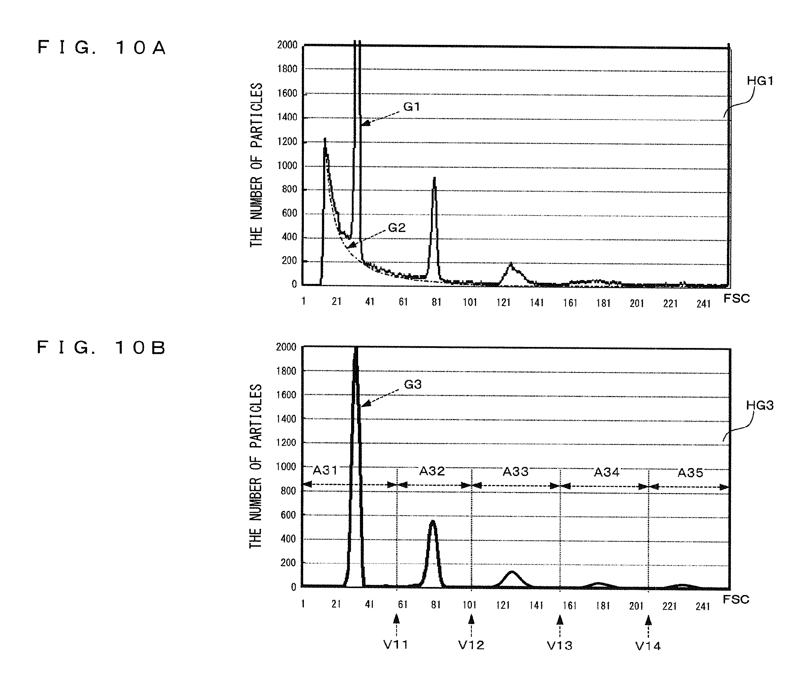

The controller 3 estimates a curve G2 showing the distribution of the non-target substances based on the histogram HG1 shown in FIG. 10A (S112). The histogram HG1 shown in FIG. 10A is the same as the histogram HG1 shown in FIG. 6A. Specifically, based on the curve G1 shown in FIG. 10A, the controller 3 estimates the curve G2 showing the distribution of the non-target substances by use of a spline function. Subsequently, the controller 3 creates a histogram HG3 shown in FIG. 10B by subtracting the curve G2 from the curve G1, and sets a curve G3 indicating the distribution of the target particles (non-agglutinated particles and agglutinated particle) (S113). Such a method for setting the curve G3 is described in detail in U.S. Patent No. 5527714.

Subsequently, based on the values V11 to V 14 (see FIG. 6B) calculated in S 15 in FIG. 4, the controller 3 demarcates the histogram HG3 in which the curve G3 has been set, and sets regions A31 to A3 5 as shown in FIG. 10B (S114).

Next, the controller 3 sets 1 to the variable i (S115). Subsequently, the controller 3 counts the particles (particles whose binding number is i) included in region A3i, and obtains the count value as the second count value C2i (S116). Here, since the value of the variable i is 1, the count value of the particles included in the region A31 is obtained as the second count value C21. Then, the controller 3 adds 1 to the variable i (S117). When the value of the variable i is less than or equal to 5 (S118: NO), the controller 3 returns the processing to S 116 and performs the process for obtaining the second count value C2i for the region A3i. Here, since the value of the variable i is 2 as a result of the process of S117, a process for obtaining the second count value C22 for the region A32 is performed. The process for obtaining the second count value C2i is repeated until the value of the variable i exceeds 5 (S118: YES). Then, the processes of S116 and S117 are sequentially performed on the regions A31 to A35, and with respect to the particles whose binding numbers are 1 to 5, the second count values C21 to C25 are obtained respectively.

With respect to the processes S112 to S116 in FIG. 7, in S113, the curve G2 indicating the distribution of the non-target substances is subtracted from the curve G1 on the histogram HG1, whereby the histogram HG3 is created. Thus, the second count value C2i obtained by counting the particles included in its corresponding region A31 to A35 set in the histogram HG3 is a value for which influence of the non-target substances is suppressed. In this case, as shown in FIG. 10A, in regions in which FSC values are small, the number of non-target substances is large. Thus, the distribution of the non-target substances can be estimated with high accuracy. Accordingly, the accuracy of removing data of the non-target substances by the second removing process (S112 to S114) is increased in regions where FSC values are small.

However, as shown in FIG. 10A, in accordance with increase of the FSC value, the number of the non-target substances gradually approaches zero, and thus, in regions in which FSC values are large, it becomes difficult to estimate the distribution of the non-target substances with high accuracy. Therefore, the accuracy of removing data of the non-target substances by the second removing process (S 112 to S114) gradually decreases in accordance with increase of the FSC value.

Therefore, the accuracy of removing data of the non-target substances by the second removing process (S112 to S 114) is high for regions (regions A31, A32, and the like) with small binding numbers, but decreases for regions (regions A3 4, A35, and the like) with large binding numbers.

In contrast, the accuracy of removing data of the non-target substances by the first removing process (S102, S103, and S108) is high in regions (regions A34, A3 5, and the like) with large binding numbers, in reverse of the second removing process (S112 to S 114), and low in regions (regions A31, A32, and the like) with small binding numbers. Accordingly, it is preferable that, for regions in which the accuracy of the first removing process is high, i.e., regions whose flag Fi is 1, the first removing process is employed, and for regions in which the accuracy of the first removing process is low, i.e., regions whose flag Fi is 2, the second removing process is employed.

Thus, in the present embodiment, either one of the first count value C1i obtained through the first removing process (S102, S103, and S108) and the second count value C2i obtained through the second removing process (S112 to S114) is selected as the count value for the particles whose binding number is i, based on the value of the flag Fi.

FIG. 8A is a flow chart showing the selecting process. The flow chart in FIG. 8A is continued from S 118 in FIG. 7.

The controller 3 sets 1 to the variable i (S 119), and determines whether the value of the flag Fi set in S 106 or S 107 is 1 (S120). When the value of the flag Fi is 1 (S 120: YES), the controller 3 employs the first count value C1i as the number of particles whose binding number is i (S121), and when the value of the flag Fi is 2 (S120: NO), the controller 3 employs the second count value C2i as the number of particles whose binding number is i (S 122). In this manner, the processes of S120 to S 123 are repeated until the variable i exceeds 5 (S124). Accordingly, as the number of each type of particles whose binding number is 1 to 5, either one of the first count value C1i and the second count value C2i is employed. Then, the counting process ends.

In this manner, as the number of respective types of particles whose binding numbers are 1 to 5, either one of the first count value and the second count value is employed, based on the values of the flags F1 to F5, respectively. As a result, compared with a case where only the first count value C1i is employed as the number of all types of particles whose binding number are 1 to 5, and compared with a case where only the second count value C2i is employed as the number of all types of particles whose binding number are 1 to 5, a highly-accurate count value is employed. That is, as described above, with respect to particles whose binding number is large, the accuracy of the first removing process is high, and thus, the accuracy of the first count value C1i tends to be increased. With respect to particles whose binding number is small, the accuracy of the second removing process is high, and thus, the accuracy of the second count value C2i tends to be increased. Accordingly, for particles whose binding number is large, the first count value is preferably employed, and for particles whose binding number is small, the second count value is preferably employed. In the present embodiment, whether to employ the first count value is determined in S105, and based on the result of the determination, either one of the first or second count values is employed. Therefore, the number of particles whose binding number is 1 to 5 can be accurately obtained. Accordingly, in S18 in FIG. 4, a highly-accurate agglutination degree and a highly-accurate concentration can be calculated.

Next, results of calculation of concentration of a measurement target substance actually performed by the immunoassay apparatus 1 in accordance with the flow charts in FIG. 4, FIG. 7, and FIG. 8A will be described.

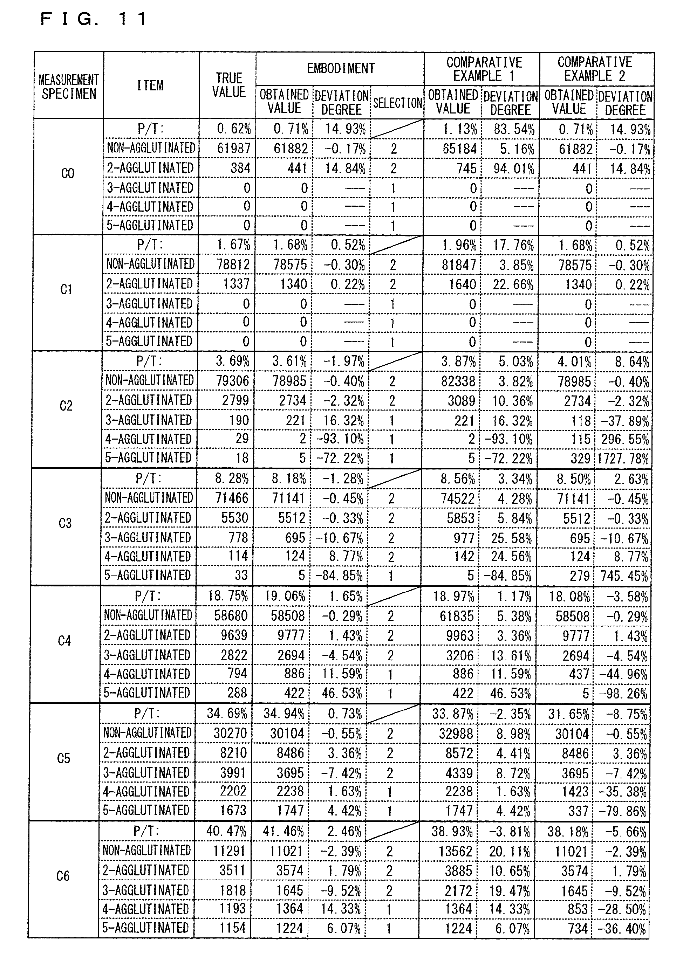

FIG. 11 shows results obtained by performing the processing (the present embodiment) shown in FIG. 4, FIG. 7, and FIG. 8A, processing (comparative example 1) in the case of obtaining only the first count values, and processing (comparative example 2) in the case of obtaining only the second count values, on seven measurement specimens C0 to C6.

In this measurement, as the measurement specimens C0 to C6, "Ranream HBsAg" produced by Sysmex Corporation was used. This is a reagent kit for HBs antigen measurement, and includes an HBsAg latex reagent, an HBsAg buffer solution, an HBsAg sample diluent, and HBsAg calibrators. In this measurement, the HBsAg calibrators were used as samples, and the HBsAg buffer solution was used as a reaction buffer solution. A carrier particle suspension was separately prepared for this measurement. Further, in this measurement, as a chyle specimen, "Interference check A plus" produced by Sysmex Corporation was used. In this measurement, a mixture of each sample, the reaction buffer solution, and the carrier particle suspension in a cuvette in the reaction part 53 was agitated for 5 minutes while being kept at 45°C. The dispensed amount of the sample was 10 µL, the dispensed amount of the reaction buffer solution was 80 µL, and the dispensed amount of the carrier particle suspension was 10 µL.

The HBsAg latex reagent is a suspension of latex particles having immobilized thereon an anti HBs antibody. The HBs antigen is a surface antigen of hepatitis B virus (HBV). Through measurement using the reagent for HBs antigen measurement, a state of HBV infection can be checked.

With reference to FIG. 11, in six "items" provided for each measurement specimen, agglutination degree (P/T), and information regarding particles (non-agglutinated particles to 5-agglutinated particles) whose binding numbers are 1 to 5 are shown individually. In "embodiment", "comparative example 1", and "comparative example 2", a result regarding a measurement specimen mixed with the chyle specimen is shown. In "true value", a result regarding a measurement specimen not mixed with the chyle specimen is shown. With respect to the embodiment and the comparative examples 1 and 2, in "obtained value", an obtained agglutination degree, and an obtained number of particle are shown; and in "deviation degree", a deviation degree from the value in "true value" is shown. In "selection" in the embodiment, which one of the comparative example 1 (first count value) and the comparative example 2 (second count value) was employed in accordance with the counting process shown in FIG. 7 and FIG. 8A is shown.

In the present embodiment, with respect to the number of each type of particles whose binding number is 1 to 5, the count value, of the comparative example 1 or 2, having the smaller absolute value of the deviation degree is employed. For example, with respect to the non-agglutinated particles of the measurement specimen C2, the deviation degree of the comparative example 1 is 3.82%, and the deviation degree of the comparative example 2 is - 0.40%. The deviation degree of the present embodiment is the one having the smaller absolute value of the deviation degree, which corresponds to the count value (78985) of the comparative example 2. Further, with respect to the 3-agglutinated particles of the measurement specimen C2, the deviation degree of the comparative example 1 is 16.32%, and the deviation degree of the comparative example 2 is -37.89%. The deviation degree of the present embodiment is the one having the smaller absolute value of the deviation degree, which corresponds to the count value (221) of the comparative example 1. In this manner, in the present embodiment, the count value, of the comparative example 1 or 2, which corresponds to the deviation degree having the smaller absolute value, i.e., the count value that is closer to the true value, is employed.

In the present embodiment, with respect to each type of particles whose binding number is 1 to 5, a count value, of the comparative example 1 or 2, that is closer to the true value is employed. Therefore, the absolute value of the deviation degree of the agglutination degree calculated from the number of particles whose binding number is 1 to 5 is less than or equal to the absolute values of the deviation degrees of the comparative examples 1 and 2. For example, with respect to the measurement specimen C1, the agglutination degree of the comparative example 1 is 1.96% and the deviation degree from the true value is 17.76%, whereas the agglutination degree of the comparative example 2 is 1.68% and the deviation degree from the true value is 0.52 %. In contrast to this, the agglutination degree of the present embodiment is 1.68%, which is the same value as that of the comparative example 2. With respect to the measurement specimen C3, the agglutination degree of the comparative example 1 is 8.56% and the deviation degree from the true value is 3.34%, whereas the agglutination degree of the comparative example 2 is 8.50%, and the deviation degree from the true value is 2.63%. In contrast to this, the agglutination degree of the present embodiment is 8.18%, and the deviation degree from the true value is -1.28%. Thus, in the present embodiment, the absolute value of the deviation degree of the agglutination degree is less than or equal to the absolute values of the deviation degrees of the comparative examples 1 and 2, that is, the accuracy of the agglutination degree is increased greater than or equal to the accuracy of the agglutination degrees of the comparative examples 1 and 2.

As shown in the value in "selection" of the present embodiment, it is found that, in each measurement specimen, the comparative example 2 (second count value) is employed for the particles whose binding number is small, and the comparative example 1 (first count value) is employed for the particles whose binding number is large. It is found that, for particle having a medium binding number, either one of the comparative example 1 (first count value) and the comparative example 2 (second count value) is selected to be employed.

According to the present embodiment, the controller 3 determines whether appropriate demarcation can be realized by the demarcation line L21 for each of the regions A11 to A15 of the scattergram SG1. Upon determining that appropriate demarcation can be realized, the controller 3 selects the first count value obtained by performing the first removing process, and upon determining that appropriate demarcation cannot be realized, the controller 3 selects the second count value obtained by performing the second removing process. Accordingly, influence of errors due to non-target substances on the agglutination degree and the concentration can be reduced more effectively.

According to the present embodiment, in the determination of S105, the controller 3 determines whether the number of particles included in the region A23 shown in FIG. 9D and the ratio of the number of particles included in the region A23 relative to the number of particles included in the entirety of the histogram HG2 are less than or equal to the predetermined threshold values Csh2 and Csh1, respectively. Through these determinations, whether the borderline between the left peak portion corresponding to the non-target substances and the right peak portion corresponding to the target particles in the histogram HG2 is clear can be appropriately determined. As a result, appropriateness/inappropriateness of the first removing process can be appropriately determined through the determination of S 105, and thus, selection between the first count value and the second count value can be appropriately performed.

In the above embodiment, the processing shown in FIG. 4, FIG. 7, and FIG. 8A has been described by using the scattergram SG1 and the histograms HG1 to HG3, and in these figures, the demarcation lines and the regions have been shown. However, as described above, these figures, the demarcation lines, and the regions are not necessarily actually rendered and displayed, for example, in the display input unit 2, but are sufficient to be set as processing performed by the analysis section 32 of the controller 3. Specifically, for example, to information of all particles stored in the storage section 31, information indicating which type, among the particles whose binding numbers are 1 to 5 and the particles of non-target substances, each particle has been classified as is added, and the demarcation lines and the regions are set by numerical values indicating coordinates.

Although an embodiment of the present invention has been described, the embodiment of the present invention is not limited thereto.

For example, in the above embodiment, with respect to all types of particles whose binding numbers are 1 to 5, the first and second removing processes are performed to obtain both of the first and second count values, and then, either one of the first and second count values is selected. However, the present invention is not limited thereto. Of the first and second count values, only an appropriate count value may be obtained.

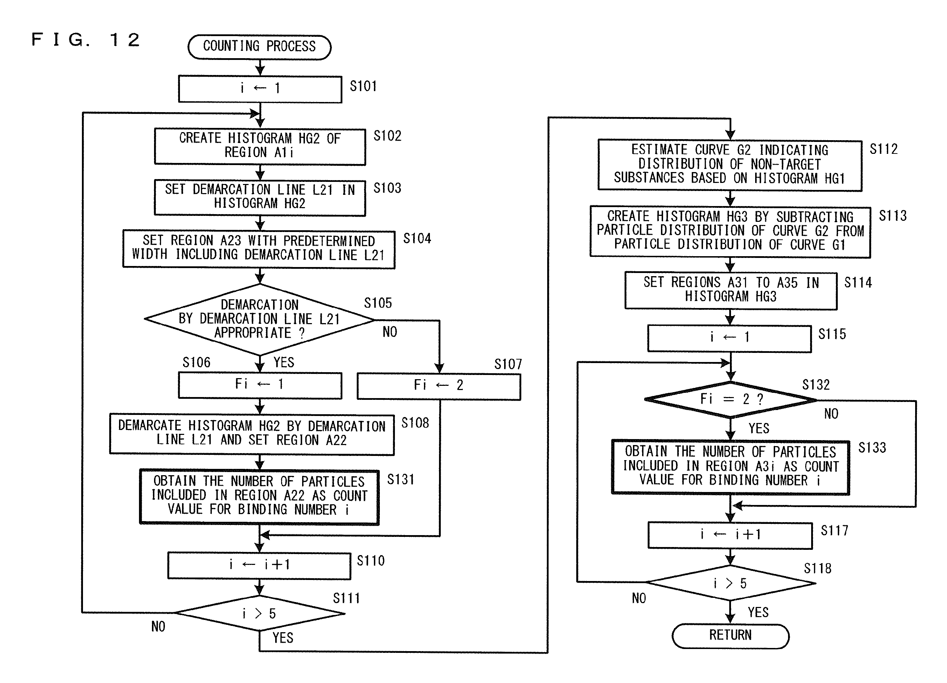

FIG. 12 is a flow chart (modification 1) showing a counting process in this case. In the counting process shown in FIG. 12, S 109 and S116 of the counting process shown in FIG. 7 and FIG. 8A are respectively replaced with S131 and S 133, and S 132 is added after S 115. Moreover, after the process of S 107, the processing is advanced to S110. Further, in the counting process shown in FIG. 12, S119 to S 124 in FIG. 8A are omitted.

In this case, with respect to the particles whose binding number is i, if the demarcation by the demarcation line L21 is not appropriate (S105: NO), the controller 3 sets 2 to the flag Fi (S107) and advances the processing to S110. That is, in the present modification, when the demarcation by the demarcation line L21 is not appropriate (S105: NO), the first removing process (S108) and the process for obtaining the count value of the number of particles (S 131) are skipped. On the other hand, with respect to the particles whose binding number is i, when the demarcation by the demarcation line L21 is appropriate (S105: YES), the controller 3 sets 1 to the flag Fi (S106), counts the number of particles included in the region A22 demarcated by the demarcation line L21, and obtains the count value as the number of particles whose binding number is i (S131).

After setting the variable i to 1 in S115, the controller 3 determines whether the value of the flag Fi is 2 (S132). When the value of the flag Fi is not 2 (S132: NO), the controller 3 skips the process for obtaining the count value of the number of particles (S133). On the other hand, when the value of the flag Fi is 2 (S 132: YES), the controller 3 counts the number of particles included in the region A3i, and obtains the count value as the number of particles whose binding number is i (S133).

According to the counting process shown in FIG. 12, as in the above embodiment, the number of particles whose binding number is 1 to 5 can be accurately obtained. When the first removing process is not appropriate (S105: NO), the first removing process (S108) is not performed, and thus, the processing can be performed efficiently and quickly. Further, when the first removing process is not appropriate (S105: NO), the process for obtaining the count value of S 131 is skipped, and when the second removing process is not appropriate (S132: NO), the process for obtaining the count value of S 133 is skipped. Therefore, compared with the above embodiment, the counting process can be efficiently and quickly performed.

Further, in the above embodiment, with respect to each of all the five types of particles whose binding numbers are 1 to 5, whether the first removing process is appropriate is determined in S105. However, without performing such determination, for each of the binding number, either one of the first removing process and the second removing process may be selected in advance as a process appropriate for removing data of the non-target substances.

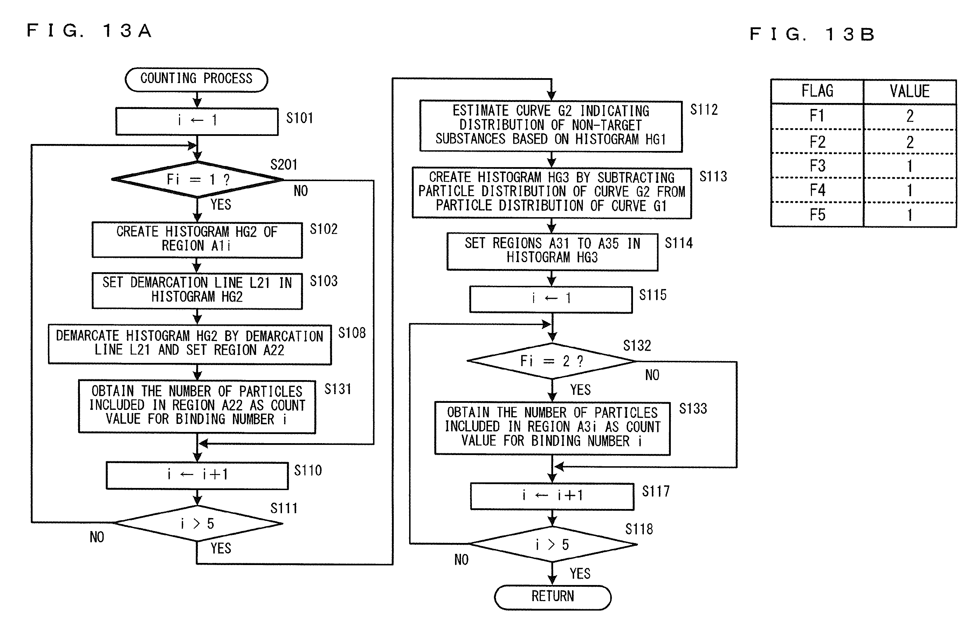

FIG. 13A is a flow chart (modification 2) showing a counting process in this case. In the counting process in FIG. 13A, S201 is added to and S103 to S106 are omitted from the counting process in FIG. 12. Further, the storage section 31 holds a table shown in FIG. 13B, and the flags F1 to F5 indicating which of the first removing process and the second removing process is selected are associated with their values. When the value of the flag Fi is 1, the first removing process (S102, S 103, and S108) is selected as the appropriate removing process, and when the value of the flag Fi is 2, the second removing process (S112 to S 114) is selected as the appropriate removing process.

The controller 3 sets 1 to the variable i (S101), and determines whether the value of the flag Fi stored in the storage section 31 is 1 (S201). Here, when the value of the flag Fi is not 1 (S201: NO), the controller 3 skips the first removing process (S102, S103, and S108) and the process for obtaining the count value of the number of particles (S131), and adds 1 to the variable i (S110). On the other hand, when the value of the flag Fi is 1 (S201: YES), the controller 3 performs the first removing process (S102, S103, and S108) to set the region A22, further counts the number of particles included in the set region A22, and obtains the count value as the number of particle whose binding number is i (S131).

The above processing is repeated until the value of the variable i exceeds 5. Accordingly, only with respect to the particles having a binding number for which the value of the flag Fi is 1, the number of particles is obtained. When the flag Fi is set as shown in FIG. 13B, only with respect to the particles whose binding number is 3, 4, or 5, the number of particles is obtained through the processes of S 102, S 103, S 108, and S131, and with respect to the particles whose binding number is 1 or 2, the number of particles is not obtained. With respect to the particle whose binding number is 1 or 2, the number of particles is obtained through the processes of S 132 and S133.

According to the counting process shown in FIG. 13A, as in the above embodiment, the number of particle whose binding number is 1 to 5 can be accurately obtained. Further, since whether the first removing process (S102, S103, and S108) is appropriate for removing data of the non-target substances is not determined, the processing can be simplified and quickly performed.

As described above, with respect to the particles whose binding number is large, the accuracy of the first removing process is high, and with respect to the particles whose binding number is small, the accuracy of the second removing process is high. Therefore, based on this tendency, it is also possible that either one of the first removing process and the second removing process is selected in advance as the removing process appropriate for removing data of the non-target substances. The contents of the setting of the flag Fi shown in FIG. 13B reflect this tendency. That is, for each of the flags F4 and F5 corresponding to particles whose binding number is large, the value 1 indicating that the first removing process is selected is set because the accuracy of the first removing process is high. For each of the flags F1 and F2 corresponding to particles whose binding number is small, the value 2 indicating that the second removing process is selected is set because the accuracy of the second removing process is high. Also from the verification results shown in FIG. 11, it is known that the accuracy of the first removing process is high for the particles whose binding number is 4 or 5, and the accuracy of the second removing process is high for the particles whose binding number is 1 or 2.

As described above, the technique of selecting and setting in advance either one of the first removing process and the second removing process is preferred, especially when it is statistically and empirically clear which of the removing processes is appropriate for particles of a certain binding number.

From the verification results shown in FIG. 11, with respect to the particles whose binding number is 3, which of the first removing process and the second removing process is appropriate for removing data of the non-target substances tends to differ depending on the measurement target specimen. In contrast, in the setting of the flag Fi shown in FIG. 13B, for the particles whose binding number is 3, the first removing process is selected. Therefore, it is conceivable that, depending on the measurement target specimen, the number of particles obtained for the particles whose binding number is 3 may include errors due to the non-target substances.

However, although the accuracy of the number of particles obtained for the particles whose binding number is 3 may be slightly reduced, the accuracy of the number of particles obtained for the particle whose binding number is other than 3 is kept high. Therefore, also by the present modification, the accuracy of the agglutination degree and the concentration calculated in S18 in FIG. 4 can be increased.

In the counting process in FIG. 13A, only with respect to the particles (here, particles whose binding number is 3) having a binding number for which it is not clear which of the first removing process and the second removing process is appropriate for removing data of the non-target substances, which of the first removing process and the second removing process is appropriate for removing data of the non-target substances may be determined.

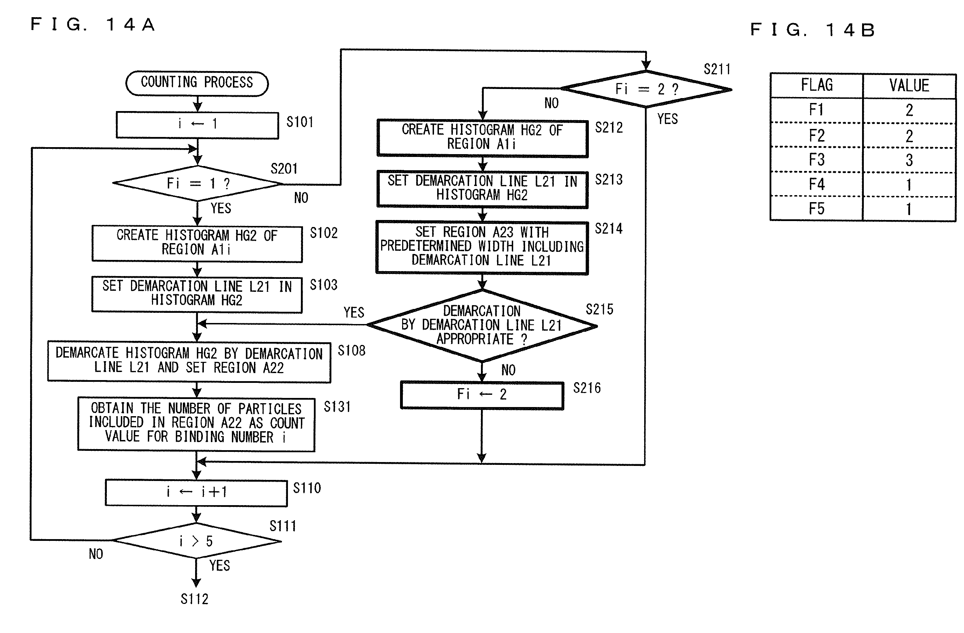

FIG. 14A is a flow chart (modification 3) showing a counting process in this case. In the flow chart shown in FIG. 14A, the process steps of S111 and thereafter are the same as those in the flow chart shown in FIG. 13A. In the counting process in FIG. 14A, S211 to S216 are added to the counting process in FIG. 13. Further, the storage section 31 holds a table shown in FIG. 14B, and the flags F1 to F5 indicating which of the first removing process and the second removing process is selected or indicating that a determination process is performed are associated with their values. When the value of the flag Fi is 1, the first removing process (S 102, S 103, and S 108) is selected as the appropriate removing process. When the value of the flag Fi is 2, the second removing process (S 112 to S 114, see FIG. 13A) is selected as the appropriate removing process. When the value of the flag Fi is 3, a determination process for determining which of the first removing process and the second removing process is appropriate for removing data of the non-target substances is selected.

In S201, upon determining that the value of the flag Fi is not 1 (S201: NO), the controller 3 further determines whether the value of the flag Fi is 2 (S211). When the value of the flag Fi is 2 (S211: YES), the controller 3 advances the processing to S110. On the other hand, when the value of the flag Fi is not 2, that is, when the value of the flag Fi is 3 (S211: NO), the controller 3 performs the determination process of S212 to S215 on the region A1i. The processes of S212 to S215 are the same as the processes of S 102 to S 105 in FIG. 7, respectively.

In S215, upon determining that the demarcation by the demarcation line L21 is appropriate, that is, upon determining that the first removing process is appropriate for removing data of the non-target substances (S215: YES), the controller 3 advances the processing to S108 and performs the process for obtaining the count value of the number of particles for the region A1i (here, the region A13) (S108, S131). On the other hand, upon determining that the demarcation by the demarcation line L21 is not appropriate, that is, upon determining that the first removing process is not appropriate for removing data of the non-target substances (S215: NO), the controller 3 temporarily rewrites the flag Fi to 2 (S216), and advances the processing to S110. In this case, with respect to the particles whose binding number is i (here, particles whose binding number is 3), the determination in S 132 in FIG. 13A becomes YES, and the process for obtaining the count value of S 133 is performed, and the number of particles based on the second removing process is obtained.

According to the counting process shown in FIG. 14A, as in the above embodiment, the number of particles whose binding number is 1 to 5 can be accurately obtained. Further, only with respect to the particles having a binding number for which it is not clear which of the first removing process and the second removing process is appropriate for removing data of the non-target substances, the determination process is performed. Thus, while increasing the accuracy of counting the number of particles, the processing can be simplified and quickly performed.

As described above, the process of determining which of the first removing process and the second removing process is appropriate for removing data of the non-target substances is preferred, especially when it is statistically and empirically not clear which of the removing processes is appropriate for the particles of a certain binding number.

Further, the immunoassay apparatus 1 may be configured such that, in a case where the counting process is performed as shown in FIG. 14A, a plurality of the tables (the values of the flags F1 to F5) shown in FIG. 14B are stored in the storage section 31, and the tables can be switched as appropriate in accordance with the measurement target substance, the measurement condition, and the like. Accordingly, even when the measurement target substance, the measurement condition, or the like is changed, the number of particles can be accurately obtained.

In the above embodiment, FSC is used as the horizontal axis of the scattergram SG1 and the horizontal axes of the histograms HG1 and HG3. However, instead of FSC, electrical information may be used such as a direct current resistance obtained when a particle passes between electrodes between which a direct current flows. Further, SSC is used as the vertical axis of the scattergram SG1 and the horizontal axis of the histogram HG2. However, instead of SSC, electrical information may be used such as a high-frequency resistance obtained when a particle passes between electrodes between which a high frequency current flows.

In addition to the above, various modifications of the embodiment of the present invention may be made as appropriate without departing from the scope of the technical idea defined by the claims.

## Claims

1. An immunoassay method comprising:
detecting, by measuring a measurement specimen obtained by mixing a sample and carrier particles together, information regarding a binding number of the carrier particles included in the measurement specimen, the carrier particles having immobilized thereon an antibody or an antigen against a measurement target substance;
classifying, based on the information regarding the binding number, particles included in the measurement specimen into groups, the groups being classified in accordance with binding numbers; and
obtaining information regarding an agglutination degree of the carrier particles based on data of the carrier particles obtained by performing, on at least one of the classified groups, at least one of a first removing process for removing, from a processing target, data of non-target substances different from the carrier particles, and a second removing process for removing, from the processing target, data of the non-target substances through a process different from the first removing process.

2. The immunoassay method according to claim 1, wherein
the detecting step comprises:
obtaining optical information from each particle included in the measurement specimen, by emitting light to the measurement specimen; and
detecting forward scattered light information as the information regarding the binding number, based on the obtained optical information.

3. The immunoassay method according to claim 2, wherein
the detecting step further comprises:
detecting side scattered light information based on the obtained optical information; and
the information obtaining step comprises:
in the first removing process, removing data of the non-target substances from the processing target, based on the detected side scattered light information.

4. The immunoassay method according to claim 2 or 3, wherein
the information obtaining step comprises:
in the second removing process, removing data of the non-target substances from the processing target, based on the detected forward scattered light information.

5. The immunoassay method according to any one of claims 1 to 4, wherein
the information obtaining step comprises:
selecting, for each classified group, either one of the first removing process and the second removing process as a removing process appropriate for removing data of the non-target substances.

6. The immunoassay method according to claim 5, wherein
the information obtaining step comprises:
selecting, for the classified group, either one of the first removing process and the second removing process as the removing process appropriate for removing data of the non-target substances, based on a magnitude of the binding number.

7. The immunoassay method according to claim 5 or 6, wherein
in the information obtaining step,
the first removing process is performed when the first removing process has been selected for the classified group.

8. The immunoassay method according to claim 5 or 6, wherein
the information obtaining step comprises:
performing, for each classified group, both of the first removing process and the second removing process; and
obtaining information regarding the agglutination degree of the carrier particles, based on data of the carrier particles obtained by performing the removing process selected as the removing process appropriate for removing data of the non-target substances.

9. The immunoassay method according to any one of claims 1 to 8, wherein
in the information obtaining step,
with respect to a predetermined group among the classified groups, either one of the first removing process and the second removing process is selected in advance as a removing process appropriate for removing data of the non-target substances.

10. The immunoassay method according to any one of claims 5 to 9, wherein
the information obtaining step comprises:
determining, with respect to a predetermined group among the classified groups, which of the first removing process and the second removing process is appropriate for removing data of the non-target substances; and
selecting, based on a result of the determination, either one of the first removing process and the second removing process as the removing process appropriate for removing data of the non-target substances.

11. The immunoassay method according to claim 5, wherein
in the information obtaining step,
a determination condition for determining whether the first removing process is appropriate for removing data of the non-target substances is set in advance,
the first removing process is selected for a group satisfying the determination condition, and
the second removing process is selected for a group not satisfying the determination condition.

12. The immunoassay method according to claim 10 or 11, wherein
the information obtaining step comprises:
in the first removing process, setting, for each classified group, a borderline for separating data of the carrier particles from data of the non-target substances, based on predetermined feature information representing a particle feature quantity; and
determining, for each classified group, whether the first removing process is appropriate for removing data of the non-target substances, based on a number of particles containing the feature information in a predetermined range including the borderline.

13. The immunoassay method according to claim 12, wherein
the information obtaining step comprises:
determining, when a ratio of the number of particles containing the feature information in the predetermined range relative to a number of particles included in the classified group is less than or equal to a predetermined threshold value, the first removing process to be appropriate for removing data of the non-target substances.

14. The immunoassay method according to claim 12 or 13, wherein
the detecting step comprises:
obtaining optical information from each particle included in the measurement specimen, by emitting light to the measurement specimen; and
obtaining side scattered light information as the feature information, based on the obtained optical information.

15. An immunoassay apparatus comprising:
a detector configured to detect, by measuring a measurement specimen obtained by mixing a sample and carrier particles together, information regarding a binding number of the carrier particles included in the measurement specimen, the carrier particles having immobilized thereon an antibody or an antigen against a measurement target substance; and
a controller configured to perform operations comprising:
classifying, based on the information regarding the binding number, particles included in the measurement specimen into groups, the groups being classified in accordance with the binding numbers;
performing, on at least one of the classified groups, at least one of a first removing process for removing, from a processing target, data of non-target substances different from the carrier particles, and a second removing process for removing, from the processing target, data of the non-target substances through a process different from the first process; and
obtaining information regarding an agglutination degree of the carrier particles, based on data of the carrier particles obtained by performing the at least one removing process.
